# EUROPEAN PATENT APPLICATION

(11) **EP 4 066 763 A2**
(43) Date of publication of application: **05.10.2022**
(21) Application number: 20851318.4
(22) Date of filing: 25.11.2020
(51) Int. Cl.: A61B 17/88, A61B 17/80

(54) **INSTALLATION FOR FORMING OSTEOSYNTHESIS PLATES**

(30) Priority: 27.11.2019 ES 201931954 U
(71) Applicant: Administración General de la Comunidad Autónoma de Euskadi, 01010 Vitoria-Gasteiz, Álava (ES)
(72) Inventor: GARCÍA FERNÁNDEZ, Rubén, 01010 Vitoria-Gasteiz (Alava) (ES); MARTÍN LARRAÑAGA, Nerea, 01010 Vitoria-Gasteiz (Alava) (ES)
(74) Representative: Pons
(86) International application number: PCT/ES2020/070738
(87) International publication number: WO 2021/105540

(57) **Abstract**

Installation and method for forming osteosynthesis plates with a curvature adapted to the particular bone structure of a patient, comprising a malleable template (1') suitable to be moulded according to the bone structure of the patient, wherein the installation further comprises an automatable positioning machine (3) which in turn comprises vertical rods (4) and stops (5) threaded to each of the vertical rods (4), adapted to be displaced vertically through said vertical rods (4), and press elements for pressing the osteosynthesis plate (1) and adapting it to the morphology of the malleable template and consequently to the bone structure of the patient.

## Description

### OBJECT OF THE INVENTION

The present invention relates to an installation and a method of using said installation for forming osteosynthesis plates with a curvature adapted to the particular bone structure of a patient. More particularly, an automatable positioning machine is described, which comprises a plurality of vertical rods adapted to house an osteosynthesis plate with a plurality of holes, further comprising elements for horizontal displacement through respective coordinate axes X and Y, and stops which are threaded and vertically movable to said rods, such that by means of using a malleable template previously adapted to the bone structure of the patient or preformed with a 3D biomodel of the very patient, it enables the definitive osteosynthesis plate to be formed with a precise morphology, speeding up execution times in the operating room, and further achieving greater precision, thus eliminating possible complications that may affect the quality of life of the patient.

### BACKGROUND OF THE INVENTION

The reduction (repositioning in fractures) of bone structures through osteosynthesis plates is a widely used practice in traumatology, maxillofacial surgery, thoracic surgery, cardiac surgery or plastic surgery.

Through this technique, an attempt is made to form said osteosynthesis plates by adapting them as closely as possible to the bone structure of each patient, for the subsequent fastening thereof through screws.

In the processes known in the state of the art, a malleable template is moulded to the morphology of the bone area of the particular patient to be treated, and the definitive osteosynthesis plate is moulded or formed based on this malleable template. However, it involves wasting a lot of time during the intervention, and sometimes it is difficult and/or dangerous to access the area to be reconstructed, for which reason it limits the final touch-up of the plates.

In some cases, in order to minimise these surgery times, a mirror image of a biomodel of the healthy anatomical structure is developed, which enables the osteosynthesis plate to be exactly preformed to the anatomy prior to surgery. This process is carried out one day before the surgery in order to have these sterile plates on the day of the intervention.

Likewise, this process becomes a manual and laborious work which is performed with conventional tools such as, for example, by means of Stillson wrenches or special benders. When the 3D biomodel is not available prior to surgery, this process is performed with the patient already open and can take on average even over an hour, depending on the number of plates to be formed. Consequently, these execution times affect the anaesthesia delivered to the patient, the risk of infection and other associated complications.

Said osteosynthesis plate, once it has been formed, is screwed into the bone structure in order to proceed with the reconstruction. Consequently, the adaptation thereof depends on the ability and experience of the surgeon, and small tolerances and imperfections certainly end in complications that affect the quality of life of the patient.

Pelvic surgeries are long, complex interventions with the risk of important complications, even vital ones, which condition a high level of concentration, tension and exhaustion in both surgeons and anaesthetists.

In other cases, based on the CT scan of the patient, a customised plate is made. The disadvantages are:
- It is not malleable in the operating room
- It has a high cost
- The design and manufacture require times that are not available in an emergency surgery with these features.

### DESCRIPTION OF THE INVENTION

The present invention seeks to solve some of the problems mentioned in the state of the art. More particularly, an installation for forming osteosynthesis plates with a curvature adapted to the particular bone structure of a patient is described, comprising a first malleable template suitable to be moulded according to the bone structure of the patient, commercial osteosynthesis plates comprising a plurality of holes, and which further comprises an automatable positioning machine which in turn comprises:
- a plurality of vertical rods configured to house the osteosynthesis plates and the malleable templates through the holes,
- stops threaded to each of the vertical rods, adapted to be displaced vertically through said vertical rods,
- first displacement elements adapted to horizontally displace each of the vertical rods through a first coordinate axis Y,
- second displacement elements adapted to horizontally displace each of the vertical rods through a second coordinate axis X.
- press elements for pressing the osteosynthesis plate in a final position adapted to the morphology of the malleable template and consequently to the bone structure of the patient.

Preferably, the first displacement elements comprise two first horizontal shafts which are joined integrally and perpendicularly to each of the vertical rods, wherein each one comprises a toothing and is coupled with a crown adapted to internally mesh with said teeth, thus enabling a horizontal movement of each of the vertical rods when said crown rotates through the first coordinate axis, and which further comprises a pinion adapted to simultaneously mesh with each crown of the first shafts, said pinion being intended to be rotated by a first drive element.

The second displacement elements can have a plurality of second horizontal toothed shafts, which are static and perpendicular to the first shafts, each of said second shafts being adapted to mesh with respective toothed wheels which are joined and integrally connected to the vertical rods, thus enabling a movement of each of the vertical rods in the longitudinal direction of the second shafts as each toothed wheel rotates by means of second drive elements.

In this manner, the vertical rods can be precisely displaced through all the coordinate axes. Note that the displacement means for each of the coordinate axes could be robotic, mechanical arms, hydraulic or any other displacement means that enable each rod to be moved individually with precision in each of the coordinate axes.

Preferably, the positioning machine can be automated by means of motors for both horizontal displacement elements, which are controlled and programmed by a programmable robot.

In a second aspect, the present invention describes a method which makes use of the previously described installation, wherein said method comprises, at least, the steps of:
A) moulding the malleable template until achieving the precise curvature adapted to the bone structure of the patient,
B) inserting said malleable template into the vertical rods of the positioning machine through the first holes and displacing each vertical rod to the position of the malleable template by means of the vertical displacement of the stops,
C) executing the horizontal displacement of said rods with respect to the first coordinate axis and with respect to the second coordinate axis making use of the first displacement elements and the second displacement elements,
D) recording the final position of the malleable template in step B and removing said malleable template,
E) inserting the osteosynthesis plate into the vertical rods through the holes of said osteosynthesis plate,
F) guiding displacement elements to the position registered in step C and pressing by using the press elements, thus achieving in said osteosynthesis plate the precise curvature adapted to the bone structure of the patient.

### DESCRIPTION OF THE DRAWINGS

As a complement to the description provided and for the purpose of helping to make the features of the invention more readily understandable, in accordance with a practical preferred exemplary embodiment thereof, said description is accompanied by a set of drawings which, by way of illustration and not limitation, represent the following:
Figure 1A shows a perspective view according to a preferred embodiment of the invention, wherein it shows the osteosynthesis plate which comprises a plurality of holes adapted to house screws and to be inserted into the vertical rods.
Figure 1B shows a perspective view according to a preferred embodiment of the invention, wherein it shows the malleable template which comprises a plurality of holes adapted to be inserted into the vertical rods.
Figure 2 shows a front view of the positioning machine according to a preferred embodiment of the invention wherein it shows the vertical rods and the vertical displacement stops.
Figure 3 shows a side view of the positioning machine according to the preferred embodiment described in figure 2, wherein it shows the first two shafts with the respective crowns, and the intermediate pinion adapted to mesh with said crowns in order to enable the horizontal movement in a first coordinate axis Y.
Figure 4 shows a front view of the positioning machine according to the preferred embodiment of figure 3, wherein it shows one of the second toothed horizontal shafts and the toothed wheel which meshes with said second toothed shaft, in order to enable the horizontal movement through a second coordinate axis X.

### PREFERRED EMBODIMENT OF THE INVENTION

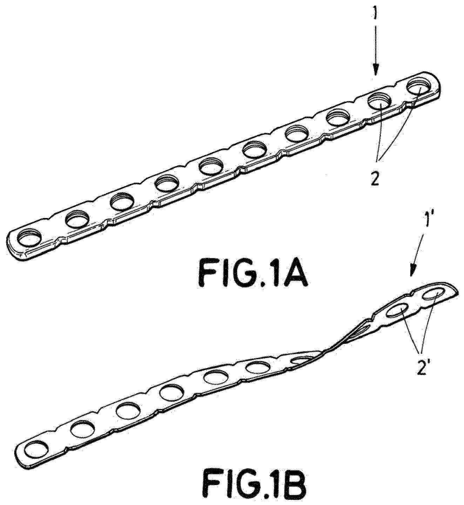

Next, with the aid of figures 1 to 4, a preferred embodiment will be described of the automatable installation for forming osteosynthesis plates (1) with a curvature adapted to the particular bone structure of a patient.

Figure 1A shows a perspective view wherein it clearly shows the osteosynthesis plate (1) prior to the adaptation to the bone anatomy of the patient, wherein said osteosynthesis plate (1) comprises a plurality of holes (2) adapted to house screws during the osteosynthesis intervention.

Figure 1B shows a perspective view according to the preferred embodiment being described, wherein it describes a malleable template (1') comprising a plurality of holes (2').

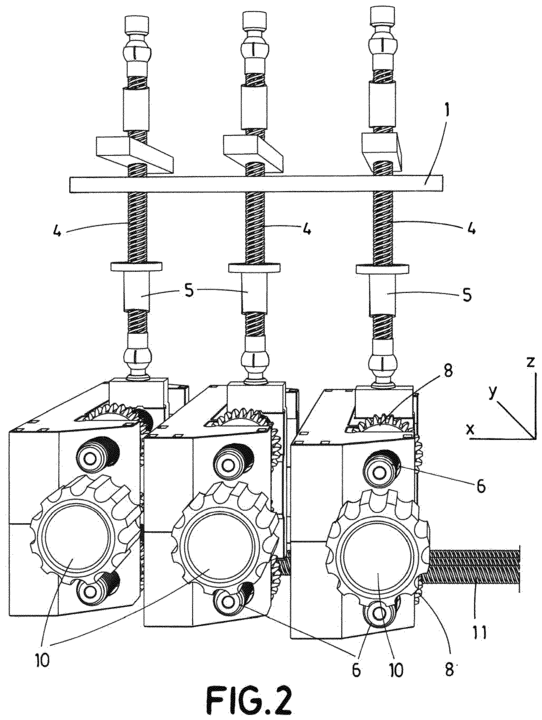

Figure 2 shows a front view of the automatable positioning machine (3) wherein it clearly shows three vertical rods (4) configured to house the osteosynthesis plates (1) and the malleable templates (1') through each of the holes (2, 2').

Likewise, figure 2 also shows that the positioning machine (3) comprises stops (5) threaded to each of the vertical rods (4), which are adapted to be displaced vertically through said vertical rods (4).

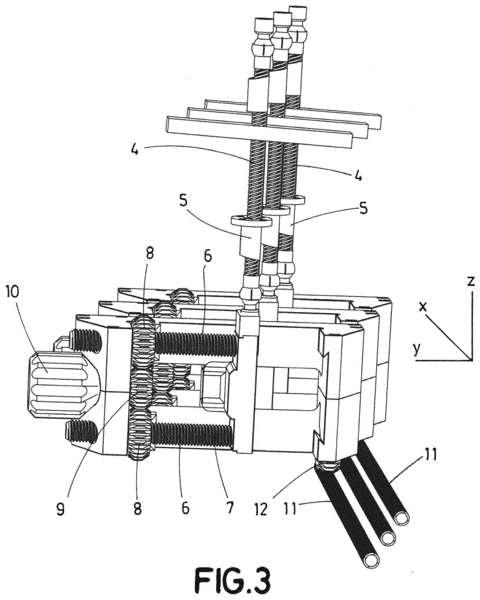

Figure 3 shows a side view of the positioning machine (3) according to the preferred embodiment described above, wherein it clearly shows horizontal displacement elements along the coordinate axis (Y) according to a preferred embodiment of the invention, wherein it shows that said elements comprise two first horizontal shafts (6) which are joined integrally and perpendicularly to each of the vertical rods (4), wherein each one comprises a toothing (7) and has a crown (8) coupled which is adapted to mesh on the inside with said toothing (7).

Consequently, it thus enables a horizontal movement through the first coordinate axis (Y) of each of the vertical rods (4) when said crown (8) is rotated.

Likewise, it also shows that the horizontal displacement elements along the axis (Y) comprise a pinion (9) adapted to simultaneously mesh with each crown (8) of the first shafts (6), said pinion (9) being intended to be rotated by a first drive element (10).

Said drive element (10) according to a preferred embodiment is a motor controlled by a programmable robot.

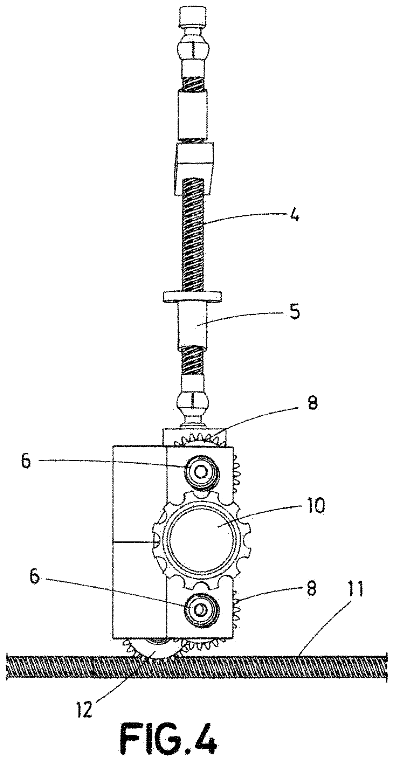

Figure 4 shows a front view of the positioning machine according to the preferred embodiment described, wherein second horizontal displacement elements are observed for displacing said vertical rods (4) along the coordinate axis X, wherein said second displacement elements comprise a plurality of second horizontal shafts (11) which are toothed, static and perpendicular to the first shafts (6), each of said second shafts (11) being adapted to mesh with corresponding toothed wheels (12) which are joined and integrally connected to the vertical rods (4).

In a preferred embodiment, the toothed wheels (12) mesh with each of the shafts (11), by means of a worm or helical gear. Consequently, thus enabling a movement of each of the vertical rods (4) in the longitudinal direction of the second shafts (11), in other words of the coordinate axis Y, as each toothed wheel (12) rotates by means of second drive elements.

Said second drive elements, according to a preferred embodiment, comprise motors, controlled by means of programmable robots.

In a second aspect, the present invention describes a method which makes use of the previously described installation, wherein said method comprises, at least, the steps of:
A) moulding the malleable template (1') until achieving the precise curvature adapted to the bone structure of the patient,
B) inserting said malleable template (1') into the vertical rods (4) of the positioning machine (3) through the first holes (2') and displacing each vertical rod (4) to the position of the malleable template (1') by means of the vertical displacement of the stops (5),
C) executing the horizontal displacement of said rods (4) with respect to the first coordinate axis (Y) and with respect to the second coordinate axis (X) making use of the first displacement elements and the second displacement elements,
D) recording the final position of the malleable template in step B and removing said malleable template (1'),
E) inserting the osteosynthesis plate (1) into the vertical rods (4) through the holes (2) of said osteosynthesis plate (1),
F) guiding displacement elements to the position registered in step C and pressing by using the press elements, thus achieving in said osteosynthesis plate (1) the precise curvature adapted to the bone structure of the patient.

## Claims

1. An installation for forming osteosynthesis plates with a curvature adapted to the particular bone structure of a patient, comprising a malleable template (1') suitable to be moulded according to the bone structure of the patient and comprising a plurality of first holes (2'), commercial osteosynthesis plates (1) comprising a plurality of second holes (2), and **characterised in that** it further comprises an automatable positioning machine (3) which in turn comprises:
- a plurality of vertical rods (4) configured to house the osteosynthesis plates (1) and the malleable templates (1') through the holes (2, 2'),
- stops (5) threaded to each of the vertical rods (4), adapted to be displaced vertically through said vertical rods (4),
- first displacement elements adapted to horizontally displace each of the vertical rods (4) through a first coordinate axis (Y),
- second displacement elements adapted to horizontally displace each of the vertical rods through a second coordinate axis (X),
- press elements for pressing the osteosynthesis plate (1) in a final position adapted to the morphology of the malleable template and consequently to the bone structure of the patient.

2. The installation of claim 1, wherein the first displacement elements comprise two first horizontal shafts (6) which are joined integrally and perpendicularly to each of the vertical rods (4), wherein each one comprises a toothing (7) and has a crown (8) coupled which is adapted to mesh on the inside with said toothing (7) thus enabling a horizontal movement of each of the vertical rods (4) when said crown (8) rotates through the first coordinate axis, and wherein said first displacement elements further comprise a pinion (9) adapted to mesh simultaneously with each crown (8) of the first shafts (6), said pinion (9) being intended to be rotated by a first drive element (10).

3. The installation of claim 2, wherein the second displacement elements comprise a plurality of second horizontal toothed shafts (11), which are static and perpendicular to the first shafts (6), each of said second shafts (11) being adapted to mesh with respective toothed wheels (12) which are joined and integrally connected to the vertical rods (4), thus enabling a movement of each of the vertical rods (4) in the longitudinal direction of the second shafts (11) as each toothed wheel (12) rotates by means of second drive elements.

4. A method for forming osteosynthesis plates with a curvature adapted to the particular bone structure of a patient, which makes use of the installation according to any of claims 1-3, comprising, at least, the steps:
A) moulding the malleable template (1') until achieving the precise curvature adapted to the bone structure of the patient,
B) inserting said malleable template (1') into the vertical rods (4) of the positioning machine (3) through the first holes (2') and displacing each vertical rod (4) to the position of the malleable template (1') by means of the vertical displacement of the stops (5),
C) executing the horizontal displacement of said rods (4) with respect to the first coordinate axis (Y) and with respect to the second coordinate axis (X) making use of the first displacement elements and the second displacement elements,
D) recording the final position of the malleable template in step B and removing said malleable template (1'),
E) inserting the osteosynthesis plate (1) into the vertical rods (4) through the holes (2) of said osteosynthesis plate (1),
F) guiding displacement elements to the position registered in step C and pressing by using the press elements, thus achieving in said osteosynthesis plate (1) the precise curvature adapted to the bone structure of the patient.
